# EUROPEAN PATENT SPECIFICATION

(11) **EP 1 608 325 B1**
(45) Date of publication and mention of the grant of the patent: **10.01.2007**
(21) Application number: 04721513.2
(22) Date of filing: 18.03.2004
(51) Int. Cl.: A61Q 5/06, A61Q 5/00, A61K 8/90

(54) **ABA BLOCK COPOLYMERS FOR HAIR STYLING COMPOSITION**
ABA-BLOCKCOPOLYMERE FÜR EINE HAARSTYLING-ZUSAMMENSETZUNG
COPOLYMERES BLOCS ABA POUR COMPOSITION DE COIFFURE

(30) Priority: 01.04.2003 EP 03252063
(43) Date of publication of application: 28.12.2005
(73) Proprietor: UNILEVER PLC, London, Greater London EC4P 4BQ (GB); UNILEVER N.V., 3013 AL Rotterdam (NL)
(72) Inventor: ADAMS, Gerald, Unilever R & D Port Sunlight, Bebington, Wirral, Merseyside CH63 3JW (GB); KHOSHDEL, Ezat, Unilever R & D Port Sunlight, Bebington, Wirral, Merseyside CH63 3JW (GB); KOSINSKI, Aleksandra, Teresa, Rolling Meadows, Chicago, IL 60008 (US); KUZNITZ, Matthew F., Unilever Home & Personal Care, Rolling Meadows, Chicago, IL 60008 (US); MUIR, Karen Elizabeth Unilever R & D Port Sunlight, Bebington, Wirral, Merseyside CH63 3JW (GB); PLANT, Yvonne C., Unilever R & D Port Sunlight, Bebington, Wirral, Merseyside CH63 3JW (GB); SEPER, Jennifer M., Unilever Home & Personal Care, Rolling Meadows, Chicago, IL 60008 (US)
(74) Representative: Tansley, Sally Elizabeth
(86) International application number: PCT/EP2004/002852
(87) International publication number: WO 2004/087081

(56) References cited:
- EP-A- 1 281 395
- WO-A-01/07496
- WO-A-94/02112
- US-A- 5 721 313

## Description

### Field of the Invention

The present invention relates to hair care compositions comprising polymers, and to their use in the treatment of hair.

### Background and Prior Art

Hair styling sprays, mousses, gels, shampoos and conditioners frequently contain resins, gums and adhesive polymers to provide a variety of benefits, for example, film-forming ability, thickening, sensory properties and hair shaping and setting.

It is known that compositions containing certain known hair styling polymers can perform poorly under conditions of high humidity. Thus, hair styled with these compositions can lose its shape and any other benefits conferred by the compositions when the ambient humidity increases or the hair and/or the scalp becomes damp.

Furthermore there are many sensory negatives associated with conventional polymers as they have tendency to give a sticky feel when applied to the hair, they can make the hair feel stiff and they can leave deposits.

Polymers for use in cosmetic and personal care compositions, such as for styling hair, are usually linear or graft homo- or co-polymers which contain various monomers in an alternating, random manner.

The hydrophobic/hydrophilic character of modern styling resins is carefully balanced to produce materials that are soluble in hydroalcoholic solvents, typically 80% VOC (volatile organics content).

Block copolymers have an advantage over conventional polymers in that the polymer architecture can be controlled more readily. This is particularly important when designing polymers with segments of distinct physical and chemical properties for particular applications. WO 98/53794 discusses (AB)ₙ block copolymers, where A is a silicone block and B is a vinylic block. US 5,271,930 (Fintex Inc) describes benzoate esters of polyalkoxylated block copolymers, such as PLURONICS^{™}, for skin and hair care compositions. US 5,472,686 and US 5,660,819 (both Nippon Unicar) describe non-hydrolysing block copolymers comprising a linear polysiloxane-polyoxyalkylene block as a repeating unit in cosmetic formulations. US 5,965,115 and US 5,972,356 (both Procter & Gamble) describe the use of silicone-polyoxyalkylene copolymer surfactants for improving stability of a polyorganosilicone emulsion in personal care compositions.

It is an object of the present invention to provide polymers which may be used in cosmetic and personal care compositions, such as hair styling compositions, which exhibit advantages over the compositions of the prior art. In particular, the invention aims to provide a class of polymers for use in hair styling compositions. It is a further aim of the present invention to provide cosmetic and personal care compositions (e.g., hair styling compositions) which have relatively good solubility properties (eg, in water, an alcohol such as ethanol or another cosmetically acceptable solvent) and perform surprisingly well under conditions of high humidity without the sensory negatives mentioned above.

### Summary of the Invention

In a first aspect, the present invention provides a personal care composition comprising a polymer comprising an ABA block copolymer, wherein the A groups are polylaminoalkyl methacrylate) blocks and the B group is derived from an ethylenically unsaturated carboxylate together with a cosmetically acceptable diluent or carrier.

In another aspect, the present invention provides a cosmetic method of treating hair which comprises applying to the hair a composition according to the invention.

A further aspect of the invention is the use of a composition of the invention for the cosmetic treatment of hair.

### Detailed Description of the Invention

### ABA Block Copolymers

The polymers of the invention may simply be ABA block copolymers or they may comprise the ABA block copolymers, with further modification such as, for example, by grafting onto the ABA block copolymer or by further reaction of the ABA block copolymer.

The polymers of the invention are preferably soluble in a solvent selected from water, ethanol and mixtures thereof. Typically, the polymers are soluble in these solvents at a level of at least 1% by weight, more preferably at least 5% by weight, most preferably at least 10 % by weight, at 25°C.

A blocks may be bonded directly to B blocks, where the chemistry of the A and B blocks permits this. Alternatively, the A blocks may be bonded to the B blocks via a suitable linker group L, so that the ABA block copolymer is of the formula A-L-B-L-A or, where the B block is branched or hyper branched, B(-L-A)ₙ. Polymers of formula A-L-B-L-A and B(-L-A)ₙ fall within the meaning of the term ABA block copolymer, as used herein. Typically, L is a divalent group having a molecular weight of from 14 to 200 Daltons which links the A and B blocks, preferably via O-C, N-C or S-C bonds to the B block. Preferably L is selected from:
-R-C (O) -O-;
-R-O-C(O)-O- ;
-R-C(O)-N(R')-;
-R-O-C (O) -N (R') - ; or
-R-N(R')-C(O)-N(R")- ;
in which R is a divalent, optionally substituted, linear or branched C₁-C₁₈ hydrocarbon radical (such as C₁-C₁₂ alkylene), and

R' and R" are independently selected from monovalent, optionally substituted, linear or branched C₁₋₁₈ hydrocarbon radicals e.g., C₁-C₁₈ alkyl, such as methyl.

Preferably, the divalent linker group is a carbonylalkylene group containing from 2 to 7 carbon atoms which forms an ester linkage to the B block, such as, for example, a group of formula -C(O)-C(CH₃)₂-.

Examples of monovalent, unsubstituted radicals are alkyl radicals, such as the methyl, ethyl, n-propyl, iso-propyl, n-butyl, iso-butyl, tert-butyl, n-pentyl, iso-pentyl, neopentyl and tert-pentyl radical; alkoxy radicals, such as the methoxy, ethoxy, n-propoxy, iso-propoxy, n-butoxy, iso-butoxy, tert-butoxy, n-pentoxy, iso-pentoxy, neo-pentoxy and tert-pentoxy radicals; hexyl radicals, such as the n-hexyl radical; alkenyl radicals, such as the vinyl, allyl, 5-hexenyl, 4-vinylcyclohexyl and the 3-norbornenyl radical; cycloalkyl radicals, such as cyclopentyl, cyclohexyl, 4-ethylcyclohexyl and cycloheptyl radical; norbornyl radicals and methylcyclohexyl radicals; aryl radicals, such as the phenyl, biphenylyl, napthyl, anthryl and phenanthryl radical; alkaryl radicals, such as o-, m- and p-tolyl radical, xylyl radicals and ethylphenyl radical; and aralkyl radicals, such as the benzyl, styryl, and phenylethyl radicals.

Examples of monovalent, substituted radicals are halogenated hydrocarbon radicals, such as the chloromethyl, 3-chloropropyl, 3-bromopropyl, 3,3,3-trifluoropropyl and 5,5,5,4,4,3,3-heptafluoropentyl radical and the chlorophenyl, dichlorophenyl and trifluorotolyl radical; mercaptoalkyl radicals, such as the 2-mercaptoethyl and 3-mercaptopropyl radical; cyanoalkyl radicals, such as the 2-cyanoethyl and 3-cyanopropyl radical; aminoalkyl radicals, such as the 3-aminopropyl, N-(2-aminoethyl)-3-aminopropyl and N-(2-aminoethyl)-3-amino-(2-methyl)propyl radical; aminoaryl radicals, such as the aminophenyl radical; acyloxyalkyl radicals, such as the 3-acryloxypropyl and 3-methacryloxypropyl radical; and hydroxyalkyl radicals, such as the hydroxypropyl radical.

Preferred monovalent radicals are independently selected from unsubstituted or substituted C₁ to C₆ alkyl radicals or optionally substituted phenyl radicals, in particular methyl, ethyl, propyl or phenyl radicals.

Examples of divalent hydrocarbon radicals are linear or branched saturated alkylene radicals, such as the methylene and ethylene radical, as well as propylene, butylene, pentylene, hexylene, cyclohexylene and octadecylene radicals; alkoxyalkylene radicals such as the methoxyethylene and ethoxyethylene radical; unsaturated alkylene or arylene radicals, such as the hexenylene radical and phenylene radicals; alkarylene radicals such as the methylphenylene and ethylphenylene radical, and alkoxyarylene radicals such as the methoxyphenylene and ethoxyphenylene radical. The divalent hydrocarbon radical R can be interrupted by divalent radicals, bonded to carbon atoms on both sides, such as -O-, -C (O) O-, -O (O) C-, -CONR⁶-, -NR⁶C(O)- and -C(O)-, where R⁶ is hydrogen or a monovalent, optionally substituted, linear or branched C₁₋₁₈ hydrocarbon radical as described above.

It is preferable if in the molecular weight Mₙ of the total polymer is from 1,000 to 1,000,000, more preferably 5,000 to 500,000.

### The A Blocks

The A blocks in the copolymers of the invention are of a polymer that is derived from an ethylenically unsaturated polymerisable monomer.

By "polymerisable" is meant monomers that can be polymerised by reaction between the monomers to form an extended polymer chain which is typically linear.

By "ethylenically unsaturated" is meant monomers that contain at least one polymerisable carbon-carbon double bond (which can be mono-, di-, tri- or tetra-substituted). Either a single monomer or a combination of two or more monomers can be utilised. In either case, the monomers are selected to meet the physical and chemical requirements of the final ABA block copolymer.

The A blocks, which may be the same or different in each ABA block copolymer (but which are preferably the same) preferably have a molecular weight Mₙ in the range of from 100 to 200,000 more preferably from 1,000 to 100,000, most preferably 1,000 to 20,000.

The A groups are poly(aminoalkylmethacrylate) blocks, such as, for example, poly(2-(dimethylamino)ethyl methacrylate) blocks.

### The B Block

The B block of the polymer is derived from an ethylenically unsaturated carboxylate groups,

The B block is preferably represented by the formula:

X- [-CH₂-CY(OR)-]ₘ[-CY' (OR)-CH₂-]ₙ-X'

wherein m and n are integers independently greater than 1; Y and Y' are independently H or -CH₃; R represents a hydrocarbyl group; and X and X' independently represent substituents active for the formation of block polymers in a transition metal mediated, atom transfer polymerisation process.

R may be selected from the group consisting of: CH₃C(O)-, Y"-C (O) -, PhC (O)-, Ph-, PhCH₂- CH₃- (CH₂-CH₂-CH₂-CH₂-) , 'C (O) -, CH₃-(CH₂-CH=CH-CH₂-)eC(O)- and substituted phenyl; wherein Y" is an n- alkyl group having up to 18 carbon atoms, Ph is phenyl, and the substituted phenyl is substituted with at least one substituent selected from the group consisting of NO₂, OMe, CN, NMe₂, OH, CL, Br, and F. Preferably, R is CH₃C(O)-, that is that the b block is derived from vinyl acetate.

The X and X groups preferably have homolytically cleavable CI and/or Br bonds.

Preferably, X and X are independently selected from the group consisting of BrCH2C(0)0-; BrC(Me)2C(0)0-..
BrCH2C (O) OCH2CH2NHC (O) CMe2-; and
BrCMe2C(O)OCH2CH2NHC(O)CMe2-.
wherein Me represents CH3-.

The B block, preferably have a molecular weight Mₙ in the range of from 100 to 300,000 more preferably from 1,000 to 200,000, most preferably 1,000 to 30,000.

### Process for producing ABA block copolymers

The polymers of the present invention may be formed by a number of different routes. However, the polymers are preferably formed according to the process described in WO 01/07496 (BP Chemicals Limited)

Particularly preferred polymers of the invention are: 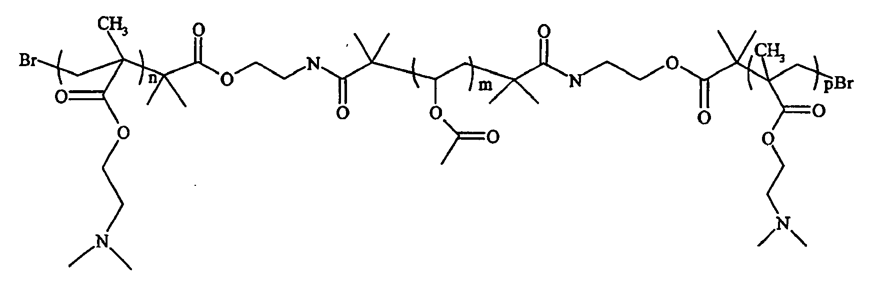
- DC17:: Total Mₙ = 28 000
where M is such that Mₙ of PVAc = 8 700
where n and p are such that Mₙ of each block of PDMAEMA = 9 700
PD = 1.34
- DC20:: Total Mₙ = 17 400
where M is such that Mₙ of PVAc = 8 700
where n and p are such that Mₙ of each block of PDMAEMA = 4 350
PD = 1.45

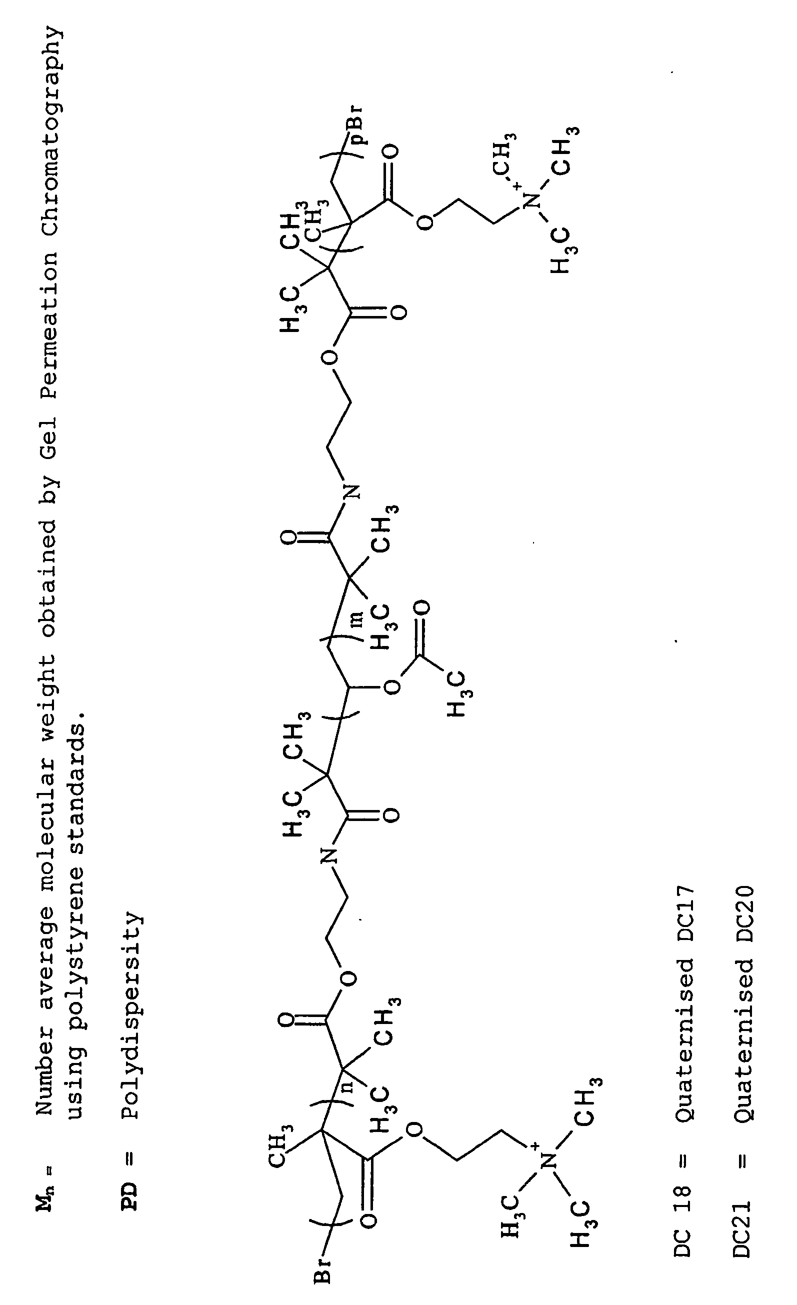

### Compositions of the invention

Cosmetic or personal care compositions of the present invention are preferably formulated into hair care compositions, especially hairspray compositions, but can also be formulated into a wide variety of product types, including mousses, gels, lotions, tonics, sprays, shampoos, conditioners, rinses, hand and body lotions, facial moisturisers, sunscreens, anti-acne preparations, topical analgesics, mascaras, and the like. Compositions of the invention comprise a cosmetically acceptable diluent or carrier. Preferably, the compositions are for use in styling human hair and, more preferably, they are packaged and labelled as such.

Compositions of the invention preferably contain the polymer in an amount of from 0.01% to 30% (more preferably from 0.1 to 10%) by weight. Compositions of the invention may, optionally, comprise a fragrance or perfume and/or one or more of the optional additional components described hereinafter.

The carriers and additional components required to formulate such products vary with product type and can be routinely chosen by one skilled in the art. The following is a description of some of these carriers and additional components.

### Carriers

Cosmetic or personal care compositions of the present invention can comprise a carrier, or a mixture of such carriers, which are suitable for application to the hair. The carriers are present at from 0.5% to 99.5%, preferably from 5.0% to 99.5%, more preferably from 10.0% to 98.0%, by weight of the composition. As used herein, the phrase "suitable for application to hair" means that the carrier does not damage or negatively affect the aesthetics of hair or cause irritation to the underlying skin.

Carriers suitable for use with hair care compositions of the present invention include, for example, those used in the formulation of hair sprays, mousses, tonics, gels, shampoos, conditioners, and rinses. The choice of appropriate carrier will also depend on the particular polymer to be used, and whether the product formulated is meant to be left on the surface to which it is applied (e.g., hair spray, mousse, tonic, or gel) or rinsed off after use (e.g., shampoo, conditioner, rinse).

The carriers used herein can include a wide range of components conventionally used in hair care compositions. The carriers can contain a solvent to dissolve or disperse the particular polymer being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Examples of suitable propellants include materials such as trichlorofluoromethane, dichlorodifluoromethane, difluoroethane, dimethylether, propane, n-butane or isobutane. A tonic or hair spray product having a low viscosity may also utilise an emulsifying agent. Examples of suitable emulsifying agents include nonionic, cationic, anionic surfactants, or mixtures thereof. If such an emulsifying agent is used, it is preferably present at a level of from 0.01% to 7.5% by weight based on total weight of the composition. The level of propellant can be adjusted as desired but is generally from about 3% to about 30% by weight based on total weight for mousse compositions and from 15% to 50% by weight based on total weight for aerosol hair spray compositions.

Suitable spray containers are well known in the art and include conventional, non-aerosol pump sprays i.e., "atomisers", aerosol containers or cans having a propellant, as described above, and also pump aerosol containers utilising compressed air as the propellant.

Where the hair care compositions are conditioners and rinses, the carrier can include a wide variety of conditioning materials. Where the hair care compositions are shampoos, the carrier can include, for example, surfactants, suspending agents, and thickeners. Hair styling creams or gels also typically contain a structurant or thickener, typically in an amount of from 0.01% to 10% by weight.

The carrier can be in a wide variety of forms. For example, emulsion carriers, including oil-in-water, water-in-oil, water-in-oil-in-water, and oil-in-water-in-silicone emulsions, are useful herein. These emulsions can cover a broad range of viscosities, e.g., from 0.1 Pa.s (100 cps) to 200 Pa.s (200,000 cps). These emulsions can also be delivered in the form of sprays using either mechanical pump containers or pressurised aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like).

### Additional Components

A wide variety of additional components can be employed in compositions according to the present invention. Examples include the following:
- hair styling polymers for hair styling compositions such as hair sprays, gels, and mousses. Hair styling polymers are well known articles of commerce and many such polymers are available commercially which contain moieties, which render the polymers cationic, anionic, amphoteric or nonionic in nature. The polymers may be synthetic or naturally derived.

The amount of the polymer may range from 0.5 to 10%, preferably 0.75 to 6% by weight based on total weight of the composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP corporation esterified copolymers of methyl vinyl ether and maleic anhydride).

Other suitable anionic hair styling polymers include carboxylated polyurethanes. Carboxylated polyurethane, resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as optional components in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from 0.001 to 10% by weight of the total composition.
- sunscreening agents such as 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof.
- anti-dandruff actives such as zinc pyrithione, piroctone olamine, selenium disulphide, sulphur, coal tar, and the like.
- hair conditioning agents such as hydrocarbons, silicone fluids, and cationic materials. The hydrocarbons can be either straight or branched chain and can contain from 10 to 16, preferably from 12 to 16 carbon atoms. Examples of suitable hydrocarbons are decane, dodecane, tetradecane, tridecane, and mixtures thereof. Examples of suitable silicone conditioning agents useful herein can include either cyclic or linear polydimethylsiloxanes, phenyl and alkyl phenyl silicones, and silicone copolyols. Cationic conditioning agents useful herein can include quaternary ammonium salts or the salts of fatty amines.
- surfactants for hair shampoo and conditioner compositions. For a shampoo, the level is preferably from 10% to 30%, preferably from 12% to 25%, by weight based on total weight of the composition. For conditioners, the preferred level of surfactant is from 0.2% to 3%, by weight based on total weight of the composition. Surfactants useful in compositions of the present invention include anionic, nonionic, cationic, zwitterionic and amphoteric surfactants.
- carboxylic acid polymer thickeners for hair shampoo and conditioner compositions. These crosslinked polymers contain one or more monomers derived from acrylic acid, substituted acrylic acids, and salts and esters of these acrylic acids and the substituted acrylic acids, wherein the crosslinking agent contains two or more carbon-carbon double bonds and derived from a polyhydric alcohol. Examples of carboxylic acid polymer thickeners useful herein are those selected from the group consisting of carbomers, acrylates/CJ.O-C30 alkyl acrylate crosspolymers, and mixtures thereof. Compositions of the present invention can comprise from 0.025% to 1%, more preferably from 0.05% to 0.75% and most preferably from 0.10% to 0.50% of the carboxylic acid polymer thickeners, by weight based on total weight of the composition.
- emulsifiers for emulsifying the various carrier components of the compositions of the invention. Suitable emulsifier types include polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, and mixtures thereof. The emulsifiers can be used individually or as a mixture of two or more and can comprise from about 0.1% to about 10%, more preferably from 1% to 7%, and most preferably from 1% to 5%, by weight based on total weight of the composition.
- vitamins and derivatives thereof (e.g., ascorbic acid, vitamin E, tocopheryl acetate, retinoic acid, retinol, retinoids, and the like).
- cationic polymers (e.g., cationic guar gum derivatives such as guar hydroxypropyltrimonium chloride and hydroxypropyl guar hydroxypropyltrimonium chloride, available as the Jaguar® series from Rhone-Poulenc).
- preservatives, antioxidants, chelators and sequestrants; and aesthetic components such as fragrances, colourings, hair nutrients and essential oils.

The invention also involves a method of styling hair by applying thereto a styling composition as is hereinabove described.

The following non-limiting Examples further illustrate the preferred embodiments of the invention. All percentages referred to are by weight based on total weight unless otherwise indicated.

### EXAMPLES

### Bond strength analysis

1µl of the 3% solids formulations were prepared using the ABA block copolymers of the invention (as described above as preferred materials) pipetted onto a hair junction. The following bond strengths were obtained.

| | | |
|---|---|---|
| Diastron MTT600 parameters: | % extension | =100 |
| | Rate (mm/min) | =10 |
| | Max force(g) | = 200 |
| | Gauge force(gmf) | = 2 |

Temperature = 25°C

| | **Ave Bond Strength (g) at 60% RH** | **Ave Bond Strength (g) at 80% RH** |
|---|---|---|
| DC17 | 36.6+/- 7.0 | 28.0+/-4.0 |
| DC18 | 10.2+/- 3.3 | 5.4+/-2.2 |
| DC20 | 38.7+/-5.8 | 32.9+/- 4.1 |
| DC21 | 8.0+/- 3.1 | 8.6+/-2.3 |

The non-quaternised version of the polymer gave superior bond strength data than the quaternised version.

### Examples 1,2 and Comparative Example A

The following pump spray compositions were prepared:

| | Example 1 | Example 2 | Example A |
|---|---|---|---|
| Polymer DC17 | 3 | | |
| Polymer DC20 | | 3 | |
| SCJ polymer | | | 3 |
| ethanol | 55 | 55 | 55 |
| water | 42 | 42 | 42 |

Polymer DC17 and polymer DC20 as described above.
SCJ polymer is a commercially available styling polymer - poly (ethyl-methacrylate-co-methacrylic acid-co-butylacrylate)

The compositions were tested by washing a wig twice with commercially available shampoo. The wig's hair is placed in velcro rollers and dried under a hood dryer for 20 minutes to set the style. The rollers are carefully removed and the initial style photographed A shield was placed over over half of the wig and the remaining half was treated with the Example by pumpimg the composition from a pump spray for 6 pumps. The shield was placed over the treated side of the wig and the second half of the wig was treated with a second Example in an identical manner. Photographs are taken throughout the study. The style retention is monitored by the stylist over a 30 minute time period under a controlled environment using the Sanyo Gallenkamp Chamber.
Conditions 21-22°C/34-35 % Humidity

| **Attribute** | **Treated** | |
|---|---|---|
| | **Head A** | **Head B** |
| | **Example 2** | **Example A** |
| **Hold** | 3 | 9 |
| Softest | 12 | 0 |
| Stickiest | 2 | 10 |
| Deposits | 2 | 10 |
| Flexible | 10 | 2 |
| Natural Movement | 12 | 0 |

Conditions 25-27°C/41-46% Humidity

| **Attribute** | **Treated** | |
|---|---|---|
| | **Head A** | **Head B** |
| | **Example 1** | **Example A** |
| **Hold** | 6 | 6 |
| Softest | 10 | 2 |
| Stickiest | 7 | 5 |
| Deposits | 3 | 9 |
| Flexible | 9 | 3 |
| Natural Movement | 9 | 3 |

### Examples 3 to 8

The following are examples of compositions according to the invention.

The materials used in the examples include the following:

| Material | Supplier | Function |
|---|---|---|
| Silicone emulsion X2 1787^{™} | Dow Corning | conditioning |
| VOLPO CS 50^{™} | Croda Chemicals | surfactant |
| Sepicide LD^{™} | Seppic | preservative |
| Cremophor RH410^{™} | BASF | stabiliser |
| Silicone DC 200/DC 24 S^{™} | Dow Corning | conditioning |
| Silwet L7602/L-720^{™} | Union Carbide | surfactant |
| CAP 40^{™} | Calor Gas | propellant |
| Carbopol 980^{™} | BF Goodrich | structurant |
| Jaguar HP-105^{™} | Rhodia | conditioning |
| Silicone Fluid 245^{™} | Dow Corning | conditioning |

Ethanol is SD Alcohol 40-B (92% active)

### Example 3

A styling mousse is formulated as follows:

| Material | % in product (w/w) |
|---|---|
| Silicone Emulsion X2 1787 | 1.2 |
| Polymer of any of Examples 1 to 2 | 1.5 |
| VOLPO CS 50 | 0.3 |
| Sepicide LD | 0.4 |
| Cremophor RH410 | 0.2 |
| Ethanol | 7.5 |
| CAP 40 | 8.0 |
| Perfume | 0.2 |
| Water | to 100% |

### Example 4

A hairspray is formulated as follows:

| Material | % in product (w/w) |
|---|---|
| Polymer of any of Examples 1 to 2 | 3.0 |
| Silicone DC200 | 0.09 |
| Silwet L7602 | 0.09 |
| CAP 40 | 35.0 |
| Ethanol | 60.0 |
| Perfume | 0.10 |
| Water | to 100% |

### Example 5

A pump spray is formulated as follows:

| Material | % w/w |
|---|---|
| Ethanol | 60.0 |
| Polymer of any of Examples 1 to 2 | 3.5 |
| Silwet L-720 | 0.3 |
| Silicone DC24S | 0.15 |
| Fragrance | 0.3 |
| Water | to 100% |

### Example 6

A styling gel is formulated as follows:

| Material | % w/w |
|---|---|
| Polymer of any of Examples 1 to 2 | 3.8 |
| Carbopol 980 | 0.4 |
| Water | to 100% |
| Sepicide LD | 0.4 |
| Sodium hydroxide (8% 2M) | 0.1 |
| Ethanol | 10.0 |
| Cremaphor RH410 | 0.4 |
| Jaguar HP-105 | 0.2 |
| Perfume | 0.15 |

### Example 7

A 55% voc propelled aerosol composition is formulated as follows:

| Material | % w/w |
|---|---|
| Polymer of any of Examples 1 to 2 | 3.75 |
| Silicone Fluid 245 | 0.20 |
| Fragrance | 0.32 |
| Ethanol | 19.53 |
| Dimethyl ether | 35.00 |
| Sodium benzoate | 0.26 |
| Cyclohexylamine | 0.21 |
| Water | to 100% |

### Example 8

A 55% voc pump hairspray composition is formulated as follows:

| Material | % w/w |
|---|---|
| Polymer of any of Examples 1 to 2 | 3.75 |
| Cyclopentasiloxane (99% active) | 0.15 |
| Benzophenone 4 | 0.0001 |
| Fragrance | 0.25 |
| Ethanol | 58.00 |
| Water | to 100% |

## Claims

1. A hair care composition comprising
i) a polymer comprising an ABA block copolymer, wherein the A groups are poly(aminoalkylmethacrylate) blocks built up and the B group is derived from an ethylenically unsaturated carboxylate and,
ii) a cosmetically acceptable diluent or carrier.

2. A personal care composition as claimed in Claim 1, wherein the A groups of the polymer are poly(2-(dimethylamino)ethyl methacrylate) blocks.

3. Personal care composition according to claim 1 or claim 2 wherein the B group of the polymer is derived from vinylacetate.

4. A personal care composition according to any preceding claim in which the molecular weight Mₙ of the total polymer is from 5,000 to 500,000.

5. A personal care composition according to any preceding claim in which the molecular weight Mₙ of each A block of the polymer is from 1,000 to 100,000.

6. A personal care composition according to any preceding claim in which the molecular weight Mₙ of each B block of the polymer is from 1,000 to 200,000

7. A personal care composition as claimed in any one of the preceding claims wherein the polymer is soluble in a solvent selected from water, ethanol and mixtures thereof.

8. A personal care composition as claimed in any preceding claim, further comprising a fragrance or perfume.

9. Composition as claimed in any preceding claim which is a hair styling composition.

10. Composition as claimed in any preceding claim which comprises from 0.1 to 10% by weight of the polymer.

11. Composition as claimed in any preceding claim further comprising an additional hair styling polymer.

12. Composition as claimed in any preceding claim, further comprising from 0.01% to 7.5% by weight of a surfactant.

13. Composition as claimed in any preceding claim, further comprising up to 30% by weight of a propellant.

14. Composition as claimed in any preceding claim which is a hair styling cream or gel including from 0.01% to 10% by weight of a structurant or thickener.

15. A cosmetic method of treating hair which comprises applying to the hair a composition according to any preceding claim.

16. Use of a composition according to any one of Claims 1 to 16 for the cosmetic treatment of hair.

## Patentansprüche

1. Haarpflegezusammensetzung, umfassend
i) ein Polymer, umfassend ein ABA-Blockcopolymer, worin die A-Gruppen aufgebaute Poly(aminoalkylmethacrylat)-Blöcke sind und die B-Gruppe aus einem ethylenisch ungesättigten Carboxylat stammt und,
ii) ein kosmetisch verträgliches Verdünnungsmittel oder Träger.

2. Körperpflegezusammensetzung nach Anspruch 1, worin die A-Gruppen des Polymers Poly(2-(dimethylamino)ethylmethacrylat)-Blöcke sind.

3. Körperpflegezusammensetzung nach Anspruch 1 oder Anspruch 2, worin die B-Gruppe des Polymers aus Vinylacetat stammt.

4. Körperpflegezusammensetzung nach einem der vorhergehenden Ansprüche, worin das Molekulargewicht Mn des gesamten Polymers 5.000 bis 500.000 beträgt.

5. Körperpflegezusammensetzung nach einem der vorhergehenden Ansprüche, worin das Molekulargewicht Mn von jedem A-Block des Polymers 1.000 bis 100.000 beträgt.

6. Körperpflegezusammensetzung nach einem der vorhergehenden Ansprüche, worin das Molekulargewicht Mn von jedem B-Block des Polymers 1.000 bis 200.000 beträgt.

7. Körperpflegezusammensetzung nach einem der vorhergehenden Ansprüche, worin das Polymer in einem Lösungsmittel, ausgewählt aus Wasser, Ethanol und Gemischen davon, löslich ist.

8. Körperpflegezusammensetzung nach einem der vorhergehenden Ansprüche, die ferner einen Aroma- oder Duftstoff umfaßt.

9. Zusammensetzung nach einem der vorhergehenden Ansprüche, die eine Haarstylingzusammensetzung ist.

10. Zusammensetzung nach einem der vorhergehenden Ansprüche, die 0,1 bis 10 Gew.-% des Polymers umfaßt.

11. Zusammensetzung nach einem der vorhergehenden Ansprüche, ferner umfassend ein zusätzliches Haarstylingpolymer.

12. Zusammensetzung nach einem der vorhergehenden Ansprüche, ferner umfassend 0,01 bis 7,5 Gew.-% eines oberflächenaktiven Mittels.

13. Zusammensetzung nach einem der vorhergehenden Ansprüche, ferner umfassend bis zu 30 Gew.-% eines Treibmittels.

14. Zusammensetzung nach einem der vorhergehenden Ansprüche, die eine Haarstylingcreme oder ein -gel ist, umfassend 0,01 bis 10 Gew.-% eines Strukturierungsmittels oder eines Verdickungsmittels.

15. Kosmetisches Verfahren zur Behandlung von Haar, umfassend das Auftragen einer Zusammensetzung nach einem der vorhergehenden Ansprüche auf das Haar.

16. Verwendung einer Zusammensetzung nach einem der Ansprüche 1 bis 16 zur kosmetischen Behandlung von Haar.

## Revendications

1. Composition de soin pour les cheveux comprenant :
i) un polymère comprenant un copolymère bloc ABA,
dans lequel les groupes A sont des blocs de poly(aminoalkylméthacrylate) accumulés et le groupe B est dérivé d'un carboxylate insaturé d'éthylène et,
ii) un diluant ou un support cosmétiquement acceptable.

2. Composition de soin personnel selon la revendication 1, dans laquelle les groupes A du polymère sont des blocs de poly(2-diméthylamino)éthyle méthacrylate).

3. Composition de soin personnel selon la revendication 1 ou 2, dans laquelle le groupe B du polymère est dérivé de l'acétate de vinyle.

4. Composition de soin personnel selon l'une quelconque des revendications précédentes, dans laquelle le poids moléculaire Mₙ du polymère total est de 5.000 à 500.000.

5. Composition de soin personnel selon l'une quelconque des revendications précédentes, dans laquelle le poids moléculaire Mₙ de chaque bloc A du polymère est de 1.000 à 100.000.

6. Composition de soin personnel selon l'une quelconque des revendications précédentes, dans laquelle le poids moléculaire Mₙ de chaque bloc B du polymère est de 1.000 à 200.000.

7. Composition de soin personnel selon l'une quelconque des revendications précédentes, dans laquelle le polymère est soluble dans un solvant sélectionné parmi l'eau, l'éthanol et les mélanges de ceux-ci.

8. Composition de soin personnel selon l'une quelconque des revendications précédentes, comprenant en plus une fragrance ou un parfum.

9. Composition de soin personnel selon l'une quelconque des revendications qui est une composition de coiffure.

10. Composition de soin personnel selon l'une quelconque des revendications précédentes qui comprend de 0,1 à 10 % en poids du polymère.

11. Composition de soin personnel selon l'une quelconque des revendications précédentes comprenant en plus un polymère de coiffure supplémentaire.

12. Composition de soin personnel selon l'une quelconque des revendications précédentes comprenant en plus de 0,01 % à 7,5 % en poids d'un tensioactif.

13. Composition de soin personnel selon l'une quelconque des revendications précédentes comprenant en plus jusqu'à 30 % en poids un propulseur.

14. Composition de soin personnel selon l'une quelconque des revendications précédentes qui est une crème ou un gel de coiffure comprenant de 0,01 % à 10 % en poids d'un agent structurant ou d'un épaississant.

15. Procédé cosmétique de traitement des cheveux qui comprend l'application sur les cheveux d'une composition selon l'une quelconque des revendications précédentes.

16. Utilisation d'une composition selon l'une quelconque des revendications 1 à 16 pour le traitement cosmétique des cheveux.
